Europäisches Patentamt

European Patent Office

Office européen des brevets

(11) Publication number: 0 383 406
A1

# EUROPEAN PATENT APPLICATION

(21) Application number: 90200345.8

(22) Date of filing: 15.02.90

(51) Int. Cl.5: A61K 9/52, A61K 9/16, A23K 1/00

(30) Priority: 15.02.89 NL 8900368

(43) Date of publication of application:
22.08.90 Bulletin 90/34

(84) Designated Contracting States:
AT BE CH DE DK ES FR GB GR IT LI NL SE

(71) Applicant: UNILEVER NV
Burgemeester s'Jacobplein 1 P.O. Box 760
NL-3000 DK Rotterdam(NL)
(84) BE CH DE DK ES FR GR IT LI NL SE AT

Applicant: UNILEVER PLC
Unilever House Blackfriars P.O. Box 68
London EC4P 4BQ(GB)
(84) GB

(72) Inventor: Van Senden, Johan Gerhard
Molenlaan 280
NL-3055 GM Rotterdam(NL)
Inventor: Sakkers, Peter Joost Dirk
Toermalijn 10
NL-3831 DC Leusden(NL)
Inventor: Roggeveen, Robert Pieter
Waterlustlaan 87
NL-2804 KX Gouda(NL)

(74) Representative: Smulders, Theodorus A.H.J.,
Ir. et al
Vereenigde Octrooibureaux Nieuwe Parklaan
107
NL-2587 BP 's-Gravenhage(NL)

(54) A process for encapsulating an active ingredient.

(57) This invention relates to a process for encapsulating an active ingredient, which comprises preparing a solution or dispersion of the active ingredient in molten fatty acid or fatty acid derivative and spraying the dispersion into a gas stream having a temperature below the melting point of the molten fatty acid or fatty acid derivative to form particles having a particle size of at least 100 μm, followed by separating the particulate encapsulated active ingredient.

**A process for encapsulating an active ingredient.**

This invention relates to a process for encapsulating an active ingredient in a fatty acid or a fatty acid derivative as well as to the resulting encapsulated active ingredient and its use in fodder.

There is a substantial need for the encapsulation of all types of active ingredients, such as perfumes and flavorings, drugs, agents beneficial to health and the like. In general, encapsulation is carried out mainly for two reasons. The first reason is to mask the flavor of the product, which is therefore basically connected with products to be swallowed by man or animal, such as drugs or the agents beneficial to health. Examples thereof are paracetamol, vitamins, amino acids, lower peptides and the like. By means of encapsulation the flavor of the active ingredient is masked, so that the product is more easily received. In particular, this may be highly important to drugs for children or animals.

When encapsulated particles are used in a syrup in which the active ingredient is a drug, the particles are preferably chosen as small as possible, because these are properly dispersible. Suitable particle sizes range from 10 to 50 $\mu$m.

The second reason for encapsulation is the protection of the active ingredient from premature release. Two variants may be distinguished, namely those situations in which the active ingredient is to be released in a controlled manner for a longer period of time (slow release) and those situations in which the active ingredient is to be released at a specific moment. For these latter situations reference may be made to, e.g., ingredients administered to ruminants and in respect of which it is important that these should not release in the rumen, but only later in the alimentary tract. These may be, e.g., ingredients that are prematurely decomposed or deactivated through the conditions in the rumen.

For the encapsulation of active ingredients a large number of methods are described in the literature.

Swiss patent 653,864 discloses a process for protecting the activity of animal feed components against the action of liquids and flora in the rumen and the stomach. This process comprises spraying a mixture of hardened fat and the active ingredient to a particle size of up to 50 $\mu$m.

This process has the drawback that only a few types of ingredients can be protected and that the protection is far from being satisfactory.

The object of the present invention is to provide a specific, very simple method for the encapsulation of active ingredients, which method leads to excellent results.

This invention therefore relates to a process for encapsulating an active ingredient, which according to the invention comprises preparing a solution or dispersion of the active ingredient in a molten fatty acid or fatty acid derivative and spraying the dispersion into a gas stream having a temperature below the melting point of the molten fatty acid or fatty acid derivative to form particles having a particle size of at least 100 $\mu$m, followed by separating the resulting particulate encapsulated active ingredient.

Surprisingly, it has been found that an excellent protection of the active ingredient is thus obtained, although as a result of the method applied a portion of the active ingredient can still be present at the surface of the particles. It has been found that the selection of the particle size of the encapsulated particles not only provides a surprisingly good protection of the active ingredient, but also the possibility to include in the encapsulated particles substantial amounts of the active ingredient. These amounts may be substantially larger than those obtained by the method according to the above Swiss patent.

According to this invention the particle size of the encapsulated particles is minimally 100 $\mu$m and will in general not be more than 5 mm, in particular not more than 2 mm. Within these limits an excellent and very effective protection is obtained, on the one hand, and, on the other hand, the strength of the particles is satisfactory, which may be a problem, especially in the case of larger sizes.

According to a special embodiment of the process according to this invention the spraying of the solution or dispersion may be followed by spraying the resulting particles in a rotary dish with molten fatty acid or fatty acid derivative. This method is known per se in the literature and is comparable to some extent with the pangranulation method known for fertilizer.

The advantage of this process particularly resides in obtaining an optimally protected active ingredient which is not even present at the surface of the particle.

The dispersion may be sprayed into the gas stream in a manner known per se by using a hydraulic sprayer or a two-phase sprayer. A person skilled in the field of spraying liquids or dispersions can determine by means of a few routine tests what sprayer is appropriate. If a two-phase sprayer is used, the temperature of the gas supplied to the sprayer is preferably adjusted to cause no clogging of the sprayer.

The dispersion is sprayed into a gas stream having a temperature below the melting point of the fatty acid or fatty acid derivative. The main reason for this temperature resides in the fact that otherwise the particles hardly, if at all, solidify, which involves the risk of agglomeration of individual particles. A certain

2

degree of agglomeration is tolerable, but too much agglomeration may lead to clogging and lumping in the further process, which is undesirable.

The gas stream may be passed both co- and countecurrently to the dispersion. In view of the fact that cooling must take place, still gas is preferably not used, because this will ultimately have an unduly high temperature. The choice of either countercurrent or cocurrent and the superficial gas velocity depend mainly on the actual process conditions. The point is that it is important for the resulting encapsulated particles not to be entrained by the gas stream.

The process according to this invention is carried out with molten fatty acid or fatty acid derivative, which term includes all types of derivatives of fatty acid, such as metal salts, esters, including mono-, di- and/or triglycerides, but also esters having 1, 2 or 4 and higher-hydric alcohols. In particular, it is preferred to use fatty acids as such or mono- and triglycerides which preferably have a melting point ranging from 30 to 120°C. More in particular, the melting point may range from 40 to 100°C, because at these temperatures the process is carried out most satisfactorily.

The choice of the fatty acid component is basically free, but the desired melting point has to·be considered. Preferred are fatty acid components based on saturated fatty acids preferably having 12-22 carbon atoms. Examples thereof are stearic acid, palmitic acid, myristic acid, lauric acid, and behenic acid. When using unsaturated fatty acids, however, it is important that the content of low-melting fatty acids is selected to cause no problems with an unduly low melting point. Consequently, more than 50% saturated fatty acid components are preferably used.

The active ingredient capable of being used according to the invention is preferably a drug, e.g., for human or animal use, or a perfume or flavoring. In this connection it should be noted that the active ingredient preferably or substantially does not comprise copper salts.

Eminently suited drugs or drugs capable of being used according to this invention as active ingredient are 5-acetylsalicylic acid or paracetamol. Encapsulation of these substances shows excellent results. Other substances to be used are, e.g., vitamins, amino acids, or lower peptides.

Another use of this invention is concerned with the encapsulation of odorants and/or flavorings. Especially in detergent compositions, such as dry abrasive powders, but also in laundry compositions, it is important that the perfume retains its effect for a long period of time, so that even after the package has long since been opened the perfume is released in a slow and metered fashion.

Depending on the nature of the use, the particle size of the encapsulated solid particles obtained by spraying can be adjusted. In general, it is possible to vary from 100 μm to 2 mm, depending on the spraying conditions. When used for animal feed, e.g., mixed in dry feed, the particle size may range from 1/10 to 2 mm.

The amount of active ingredient generally included in the encapsulated particles is preferably as large as possible. Since, however, dissolving or dispersing solids in molten fatty acid ot fatty acid derivative is limited by the viscosity of the dispersion to be sprayed, the amount of active ingredient to be included will generally be restricted. It has been found that the contents of encapsulated active ingredient in the particles may range from 1 to 45% by weight, more in particular from 5 to 25% by weight. In this connection it should be noted that it may be desirable in specific cases to include in the dispersion a wetting agent, such as a surfactant, thus enabling improvement of the dispersibility of the active ingredient in the molten fatty acid or fatty acid derivative. This admits of obtaining an improved distribution of the active ingredient over the encapsulated particles. Of course, the nature of the wetting agent depends on the selected components. In general, however, this will be a surfactant.

The present invention will now be illustrated by some examples which are intended to illustrate and not to limit the invention.


Examples 1-4

Starting from the raw materials listed in Table 1, a number of encapsulation experiments were conducted in accordance with the invention. The resulting products were then evaluated in vitro for their release pattern.

## Table 1

| | | |
|---|---|---|
| 1. | 5-aminosalicylic acid (5-ASA) | Jansen, Beerse (BE) |
| 2. | stearic acid$^x$ | Unichema Gouda (NL) |
| 3. | Tallow$^{xx}$ | Unichema Gouda (NL) |
| 4. | Glyceryl monostearate | Unichema Gouda (NL) |
| 5. | Carnauba wax | Unichema Gouda (NL) |

$^x$ iodine value < 0.2
melting point 56°C.
$^{xx}$ hardened tallow
melting point 59°C
iodine value < 2

A product with 20% 5-ASA was prepared by melting stearin at 80°C, followed by addition of 5-ASA for a period of 45 min. After homogenisation with a turbine stirrer 9 kg suspension were obtained. This suspension was sprayed into a 10 m high spraying tower. The spraying pressure was 3 bar, and the sprayer orifice was 2.55 mm.

Part of the resulting product was then coated with tallow, stearin and glycerol monostearate, respectively. The 5-ASA content of these products was 16%.

Of the resulting products the sieve reaction with a particle size of 1000-1400 µm was used.

The in vitro dissolution tests were conducted according to the paddle method (USP XXI), with sink conditions ($10 \times C_{max} < C_s$). The concentration of released 5-ASA was determined spectrophotometrically at 300 or 332 nm. The media used were simulated gastric juice pH 1.2 (USP XXI) without pepsin and simulated intestinal juice pH 6.8 and pH 7.5 (USP XXI) without pancreatin.

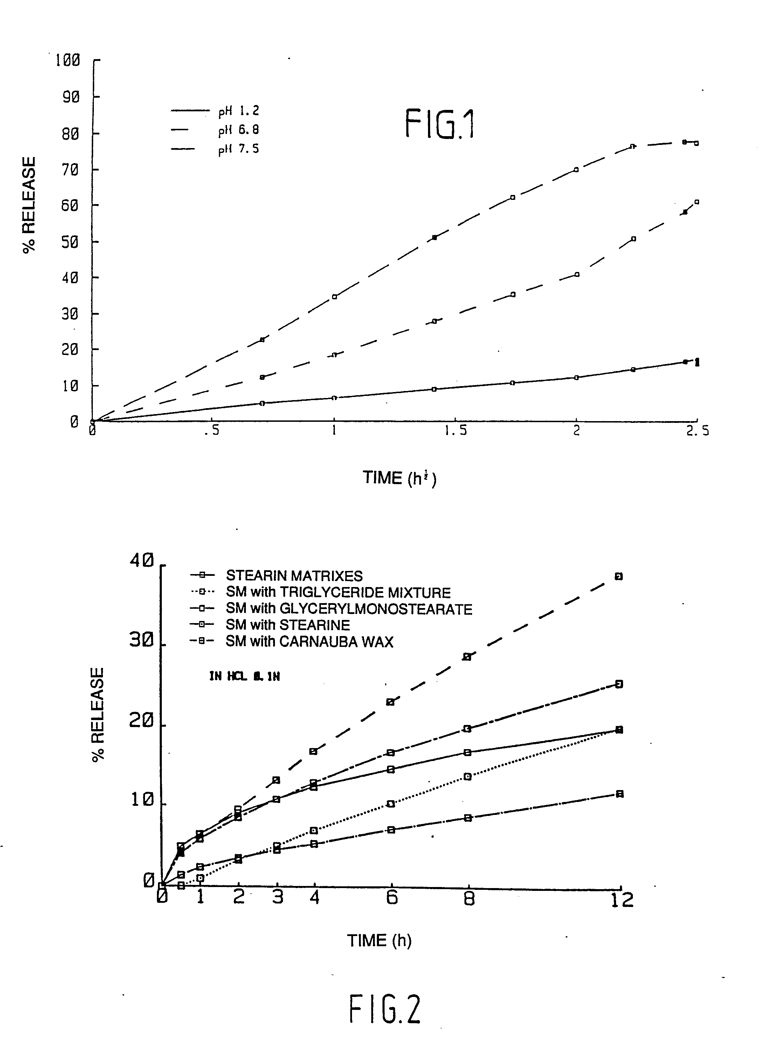

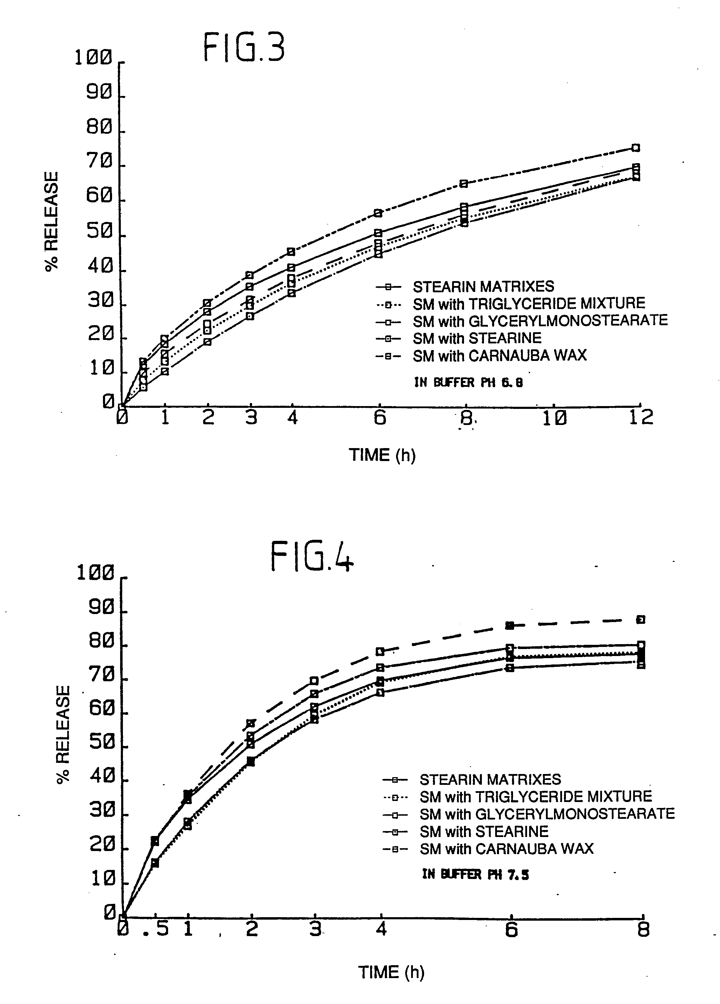

Fig. 1 shows the release profiles for the sprayed products. Figs. 2, 3 and 4 show the release profiles of the different coated sprayed products.

From the tests it appears that a good release profile is obtainable with the method according to the invention.

Example 5

35 parts of methionine were suspended in 65 parts of molten hydrogenated tallow at a temperature of 65-70°C. The resulting suspension was sprayed into a spraying tower at a spraying temperature of 65-70°C and a pressure of about $250.10^3$ Pa. The temperature at the bottom of the spraying column was 25°C. The average particle size of the particles was about 0.4 mm. A sample of 7.5 g of this product was introduced into 400 ml of an aqueous phosphate bicarbonate buffer solution with pH = 6.8 at a temperature of 37.5°C and was stirred for 6 hours in order to imitate conditions in the rumen. Then 90 ml of the solution were titrated on dissolved methionine. From this it appeared that about 60% of the methionine had passed into the buffer solution.

The remainder of the preformed particles from the spraying tower were then placed in a pan with a quickly rotating hot bottom plate (diameter 30 cm and temperature 50°C) and sprayed with 10% by weight of the above molten hydrogenated tallow. The spraying time was 40 sec, and the material was poured out after 20 sec. Again 7.5 g of the enveloped material were introduced into 400 ml buffer and stirred and then titrated in dissolved methionine. It was found that after 6 hours only 15% of the methionine present was dissolved.

Example 6

According to the technique described in Example 5, 25 parts of methionine were sprayed with 75 parts of completely hydrogenated soybean oil. The average particle size was 0.6 mm. Of these pre-formed particles it appeared after 6 hours that 45% of the methionine has passed into the buffer solution. The remainder was placed in the pan with rotating bottom plate and sprayed with 20% of molten hydrogenated soybean oil. Spraying time 2 min, temperature of the bottom plate 60°C. It appeared that of the thus enveloped feed additive only 5% of the methionine present was dissolved after stirring for 6 hours.

Example 7

According to the technique described in Example 5, 35 parts by weight of a vitamin A preparation ($10^6$ IE/g) were mixed and sprayed with 65 parts by weight of a completely hydrogenated soybean oil (spraying temperature 65-70°C, spraying pressure $2.4 \times 10^5$ Pa). The particle size of the slightly sticky material averaged 0.4 mm. Subsequently, this material was sprayed in a pan with a quickly rotating bottom plate (temperature 60°C) with 20% of molten hydrogenated soybean oil; spraying time 2 min. A vitamin A preparation stable against the action of the environment was obtained.

**Claims**

1. A process for encapsulating an active ingredient, which comprises preparing a solution or dispersion of the active ingredient in molten fatty acid or fatty acid derivative and spraying the dispersion into a gas stream having a temperature below the melting point of the molten fatty acid or fatty acid derivative to form particles having a particle size of at least 100 μm, followed by separating the particulate encapsulated active ingredient.

2. A process according to claim 1, characterized in that the active ingredient comprises animal or human drugs, or perfumes and/or flavorings.

3. A process according to claim 1 or 2, characterized in that the active ingredient not only, or substantially not only, comprises copper salts.

4. A process according to claim 3, characterized in that the active ingredient is 5-acetylsalicylic acid or paracetamol.

5. A process according to claims 1-3, characterized by selecting the active ingredient from amino acids, vitamins and lower peptides.

6. A process according to claims 1-5, in which the particles have a size of note more than 5 mm, preferably not more than 2 mm.

7. A process according to claims 1-6, in which the content of active ingredient ranges from 1 to 45%, preferably from 5 to 25%, based on the weight of the active ingredient and said fatty acid or fatty acid derivative.

8. A process according to claims 1-7, characterized by subsequently spraying the encapsulated active ingredient with molten fatty acid or fatty acid derivative.

9. A process according to claim 8, characterized by carrying out said spraying on a rotary dish.

10. A process according to claims 1-9, characterized by using fatty acid or fatty acid esters.

11. A process according to claim 10, characterized by using mono- or triglycerides.

12. A process according to claims 1-11, characterized by using fatty acid or fatty acid derivatives in which at least 50% of the fatty acid component consists of saturated fatty acids having 12-22 carbon atoms.

13. A process according to claims 1-12, characterized in that the fatty acid or fatty acid derivative has a melting point of 30-120°, preferably 40-100°C.

14. An encapsulated active ingredient obtainable by using the process according to claims 1-13.

15. The use of the encapsulated active ingredient according to claim 14 in fodder.

FIG.1

FIG.2

FIG.3

% RELEASE

- STEARIN MATRIXES
- SM with TRIGLYCERIDE MIXTURE
- SM with GLYCERYLMONOSTEARATE
- SM with STEARINE
- SM with CARNAUBA WAX

IN BUFFER PH 6.8

TIME (h)

FIG.4

% RELEASE

- STEARIN MATRIXES
- SM with TRIGLYCERIDE MIXTURE
- SM with GLYCERYLMONOSTEARATE
- SM with STEARINE
- SM with CARNAUBA WAX

IN BUFFER PH 7.5

TIME (h)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A- 922 697 (MERCK & CO.)<br>* The whole document, in particular page 3, example 6 * | 1-2,5-7 ,10-15 | A 61 K 9/52<br>A 61 K 9/16<br>A 23 K 1/00 |
| Y | | 3,4,8,9 | |
| X | US-A-3 655 864 (GRASS, Jr. et al.)<br>* Column 1, line 1 - column 5, line 39; column 3, lines 61-75 in particular * | 1-2,5-7 ,10-15 | |
| X | GB-A- 758 652 (LOVENS KEMISKE FABRIK)<br>* The whole document * | 1,2,6,7 ,10-15 | |
| X | FR-A-2 153 759 (SOGERAS)<br>* The whole document * | 1,2,6,7 ,10-15 | |
| Y | EP-A-0 284 143 (UNILEVER)<br>* The whole document * | 3,8,9 | |
| Y | DE-A-1 492 128 (SMITH KLINE & FRENCH)<br>* Pages 12-16, example * | 4 | |
| Y | WO-A-8 102 671 (FARM. LAB. FERRING)<br>* Claim 7 * | 4 | TECHNICAL FIELDS SEARCHED (Int. Cl.5)<br><br>A 61 K<br>A 23 K |
| A | CH-A- 653 864 (ALIFET AG) | | |
| A | LU-A- 73 489 (ASTRA-EWOS AB)<br>* Pages 11,12, example 5 * | 8,9 | |

The present search report has been drawn up for all claims

| Place of search | Date of completion of the search | Examiner |
|---|---|---|
| THE HAGUE | 29-05-1990 | BENZ K.F. |